(19) Europäisches Patentamt
European Patent Office
Office européen des brevets

(11) Publication number: **0 565 930 A1**

# (12) EUROPEAN PATENT APPLICATION

(21) Application number: **93105021.5**

(51) Int. Cl.[5]: **C07D 257/02**, A61K 49/00

(22) Date of filing: **26.03.93**

(30) Priority: **27.03.92 JP 102033/92**

(43) Date of publication of application:
**20.10.93 Bulletin 93/42**

(84) Designated Contracting States:
**AT BE CH DE DK ES FR GB IT LI LU NL SE**

(71) Applicant: **NIHON MEDI-PHYSICS CO., LTD.**
**9-8, Rokutanji-cho,**
**Nishinomiya**
**Hyogo(JP)**

(72) Inventor: **Kubomura, Kan**
**18-1-811, Masago,**
**3-chome**
**Mihama-ku, Chiba(JP)**
Inventor: **Seri, Shigemi**
**1157-68, Nakatakane**
**Ichihara, Chiba(JP)**
Inventor: **Iwai, Kumiko**
**35-1, Saihiro**
**Ichihara, Chiba(JP)**
Inventor: **Azuma, Makoto**
**750, Saihiro**
**Ichihara, Chiba(JP)**

(74) Representative: **von Kreisler, Alek, Dipl.-Chem. et al**
**Patentanwälte**
**Von Kreisler-Selting-Werner,**
**Postfach 10 22 41,**
**Bahnhofsvorplatz 1**
**D-50462 Köln (DE)**

## (54) Tetraazacyclododecane tetraacetic acid derivatives and the use thereof as diagnostic agents.

(57) There is disclosed a compound of the formula:

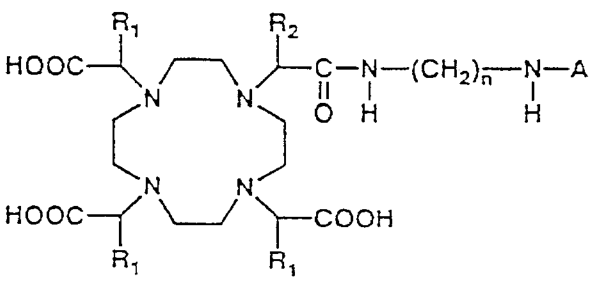

(1)

The compound (1) is a bifunctional complexing agent and forms a metal complex with a metal ion and a biologically functional material. The metal complex is useful for medical imaging diagnosis and treatment.

EP 0 565 930 A1

FIELD OF THE INVENTION

The present invention relates to a tetraazacyclododecane derivative and its use. More particularly, the tetraazacyclododecane derivative of the present invention is a novel bifunctional complexing agent having 1, 4, 7, 10-tetraazacyclododecane-4,7, 10-triacetic acid (herein after abbreviated as DO3A) skeleton, which has ability to form stable complexes with various metal ions and is useful as a carrier for metals to be applied to a living body in medical imaging diagnosis and treatment, and the like.

BACKGROUND OF THE INVENTION

In the field of medical imaging diagnosis and treatment, usefulness of safe introduction of various metal ions into a living body has been gradually established. In addition, recently, attempts have been rapidly developed to combine these metal ions with biological materials, or materials which interact with a biological system, such as proteins, peptides, antibodies, sugars, lipids and the like (herein after generally referred to as functional materials) to obtain physiological information of a living body such as reactions in a living body, for example, metabolism, enzyme reactions, receptor binding, antigen-antibody reactions and the like, or characteristic behavioral properties in a living body and the like. Then, as a technical means for coupling a metal ion to a functional material, it has been expected to use a bifunctional complexing agent as a coupling agent.

Desirable properties required for such a bifunctional complexing agent from the medical point of view vary according to a particular diagnosis and treatment. However, commonly required properties for all application are high stability of a metal ion complexed with a complexing agent and maintenance of the activity of a functional material after coupling of a complexing agent and the functional material. High stability of a complex inhibits release of the metal ion from the complexing agent and thereby minimizes side effects due to toxicity of the metal. In particular, when the metal has a long retention time in a living body, this toxicity due to the metal causes trouble. Good maintenance of the activity of a functional material can control the behavior of a metal complex compound composed of metal-bifunctional complexing agent-functional material in a living body according to a particular purpose. And, in the case of using a metal complex as an imaging agent in nuclear magnetic resonance imaging (hereinafter abbreviated as MRI) diagnosis and X-ray diagnosis, more severe properties are required for a bifunctional complexing agent. Namely, since an increased dosage in human being is needed, high water-solubility, osmotic pressure of the resulting complex near to that of body fluid of human being and ease of synthesis are required. High water-solubility make the production of a solution preparation having a high concentration possible (e.g., in the case of an imaging agent to be used for MRI diagnosis, about 0.5M). Low osmotic pressure reduces the volume of the circulatory system or the load on the body fluid balance upon administration. And, entire synthesis steps can be carried out very efficiently due to ease of synthesis, which reduces the production cost. Thus, in view of these requirements, new bifunctional complexing agents have been developed and have been variously elaborated.

As typical classic bifunctional complexing agents, so-called linear bifunctional complexing agents such as diethylenetriaminepentaacetic acid (hereinafter abbreviated as DTPA) type [JP-T 2-501385; Brechbiel M.V. et al., Inorg. Chem., 25, 2772 (1986); Esteban J.M. et al., J. Nucl. Med., 28, 861 (1987); and Westerberg D.A. et al., J. Med. Chem., 32, 236 (1989)], ethylenediaminetetraacetic acid (hereinafter abbreviated as EDTA) type [Goodwin D.A. et al., J. Med. Chem., 17, 1304 (1974); Yeh S.M. et al., Anal. Biochem., 100, 152 (1979)] and the like have been firstly developed. However, metal complex compounds obtained by using such bifunctional complexing agents tend to become instable because of acid catalytic type decomposition of complexing in serum or competitive complexing with $Ca^{2+}$ and $Zn^{2+}$, or competition with transferrin in a living body [Moerlein S.M. et al., Int. J. Nucl. Med. Biol., 8, 277 (1981)]. The lowering of stability of a complex compound promotes release of a free metal ion from the complex compound, which becomes a cause of side effects due to toxicity of the free metal ion. And, in imaging diagnosis, disordered behavior of the free metal ion causes remarkable lowering of a signal/noise ratio and an accurate information can hardly be obtained. Further, when metal ions which are apt to become trivalent ions are complexed with these complexing agents (many metal ions which are useful for medical imaging diagnosis and treatment are trivalent ions), the resulting complex compounds per se have mono- or bivalent negative charge, which results in harmful factors for a living body such as increase in osmotic pressure and the like. And, they are not always readily synthesized [Westerberg D.A. et al., J. Med. Chem., 32, 236 (1989)].

Then, recently, cyclic complexing agents, bifunctional 1, 4, 7, 10-tetraazacyclododecane-aminopolyacetic acids, have been noted as next generation compounds of the above linear bifunctional complexing agents. These complexing agents have been developed with mainly aiming at improvement of

stability of complex compounds and it has been said that, in comparison with conventional linear bifunctional complexing agents, formation of more kinetically inactive complexes with metal ions are possible. For example, A metal complex formed by gadolinium (hereinafter abbreviated as Gd) ion (III) which has been noted as a metal ion useful for MR diagnosis with 1, 4, 7, 10-tetraazacylcododecane-1, 4, 7, 10-tetraacetic acid (hereinafter abbreviated as DOTA) has much higher stability than Gd-DTPA or Gd-EDTA [Moi M.K. et al., Cancer. Res. (Suppl.), 50, 789s (1990)] and thereby acceptability to a living body is clearly improved. Therefore, the use of a bifunctional complexing agent which contains a 1, 4, 7, 10-tetraazacyclododecane-aminopolyacetic acid as its metal complexing site have a bright prospect.

1, 4, 7, 10-Tetraazacyclododecane-aminopolyacetic acid type bifunctional completing agents which have been found to be useful for medical use are generally divided into three groups. The first group is 1, 4, 7, 10-tetraacetic acid derivatives wherein a reactive side chain for participating in coupling to a functional material is bound to a ring carbon atom of the tetraazacyclododecane ring [JP-T 2-501141; Moi M.K. et al., J. Am. Chem. Soc., 110, 6266 (1988); and Deshpande S.V. et al., J. Nucl. Med., 31, 473 (1990)]. The second group is 1, 4, 7, 10-tetraacetic acid derivatives wherein carboxyl group and a reactive functional side chain for participating in coupling to a functional material are bound to the exocyclic $\alpha$-carbon atom attached to 1-nitrogen of the tetraazacyclododecane ring (EP-A 0 353 450). They are derivatives of the above DOTA and therefore they are expected to have strong complex stability with Gd ion and the like. However, due to negative charge of the resulting complex per se, increase in osmotic pressure or addition of an unnecessary counter ion in the production of a solution preparation having a high concentration are unavoidable. Further, the synthesis of the agent of the first group is generally troublesome and efficient synthesis is hardly realized because of intermolecular or intramolecular cyclization reaction. The third group is DO3A type bifunctional complexing agents which are 4, 7, 10-triacetic acid derivatives wherein 1-nitrogen of the tetraazacyclododeacne is substituted with a reactive side chain. In general, these derivatives can be readily synthesized and are advantageous in that they can form complexes having no charge. However, in comparison with DOTA type bifunctional complexing agents, complex stability is lowered and is almost the same as that of the above linear bifunctional complexing agents, DTPA derivatives. The derivatives of this third group are described in JP-A 63-41468, JP-A 64-52764; and the like. However, no special attention is paid to lowering of complex stability due to loss of one carboxyl group from a 1, 4, 7, 10-tetraacetic acid type derivative. Further, it cannot be said that the reactive functional terminal participating in coupling to a functional material is sufficiently active.

## OBJECTS OF THE INVENTION

One object of the present invention is to solve the above problems found in conventional DO3A type bifunctional complexing agents to provide a novel cyclic bifunctional complexing agent which can form a physiologically acceptable metal complex having high stability, good solubility due to no charge and a reactive functional terminal which is active and can be readily coupled to functional materials.

Another object of the present invention is to provide a novel complex compound wherein said cyclic bifunctional complexing agent is coupled to a functional material and a metal complex compound therefrom. The metal complex compound can be used as a drug in MRI diagnosis, X-ray diagnosis, radiodiagnosis or radiotherapeutics.

These objects as well as other objects and advantages of the present invention will become apparent to those skilled in the art from the following description with reference to the accompanying drawings.

## BRIEF EXPLANATION OF THE DRAWINGS

Fig. 1 is a photograph of MRI as described hereinafter showing a traverse view of the abdominal region including the liver of a mouse before administration of the compound of the present invention.

Fig. 2 is a photograph of MRI as described hereinafter showing a traverse view of the abdominal region including the liver of a mouse at about 55 minutes after administration of DNS-DOGMA-Gd solution.

## SUMMARY OF THE INVENTION

In the present invention, the bifunctional complexing agent has a reactive functional side chain bound to 1-nitrogen of DO3A as a part of its chemical structure and this side chain advantageously acts as steric hindrance to conformation cleavage of the structure of a metal complex which is required for release of a metal ion from a bifunctional complexing agent. In this regard, although there are various reactive functional side chains, any of them cannot necessarily be used for this purpose.

The present inventors have intensively studied to minimize release of a metal ion from such a bifunctional complexing agent. As a result, it has been found that, when an amide bond moiety to which a carbonyl group is bound is present in a reactive functional side chain located at $\beta$-position with respect to the 1-nitrogen atom, the desired advantageous action can be realized. Further, it has also been found that the reactive functional side chain should be have a reactive terminal functional group having sufficient activity for directly and readily reacting with a functional material to form a covalent bond. As such a reactive functional group, there can be used an active group which can readily react with a reactive group of a functional material such as thiol group, amino group, carboxyl group, hydroxyl group, aldehyde group, an aromatic group (e.g., phenyl group, etc.) or a heterocyclic group (e.g., imidazolyl group, etc.), i.e., can be readily react with a functional material without any special reagent. However, by taking into consideration of solubility, coupling property to blood protein in a living body and excretion in a urine, the reactive functional side chain should contain no straight or branched aryl group. Further, as described above, many metal ions which are useful for medical imaging diagnosis and treatment are trivalent ions (e.g., Gd, Dy, Ho, Bi, Os, In, Ga, Tc, Y, Sm, etc.) and therefore the bifunctional complexing agent of the present invention and such a metal ion can form a complex having no charge. Thereby, it is possible to prepare a solution having a high concentration with great physiological acceptability due to low osmotic pressure. The present invention has been completed based on the above finding.

Thus, according to the present invention, there is provided a physiologically acceptable compound of the formula:

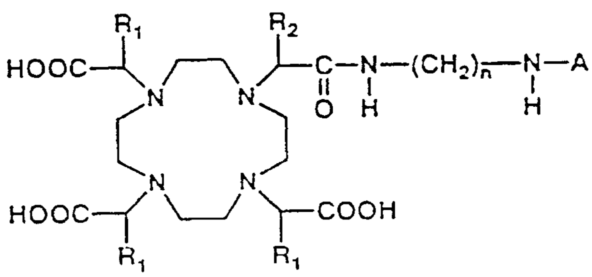

(1)

wherein n is an integer of 1 to 6; $R_1$ and $R_2$ are the same and different and are independently an hydrogen atom or a $C_{1-4}$ alkyl group; and A is an hydrogen atom or a reactive functional group having a terminal reactive group which is bound to a saturated or unsaturated, straight or branched $C_{1-4}$ alkylene group optionally interrupted with a carbonyl group [hereinafter referred to as the compound (1)], or a salt thereof.

The present invention also provides a physiologically acceptable complex compound of the formula:

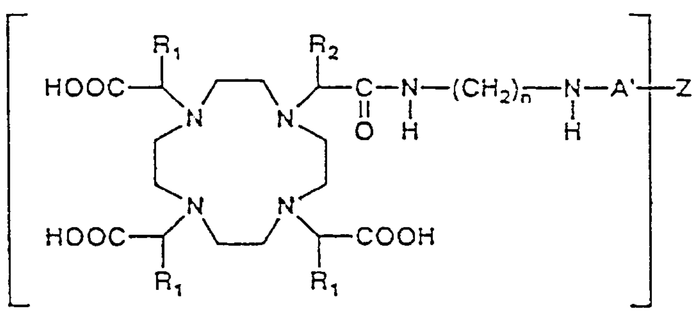

(2)

wherein n is an integer of 1 to 6; $R_1$ and $R_2$ are the same or different and are independently a hydrogen atom or a $C_{1-4}$ alkyl group; A' is a functional reactive residue having a terminal reactive group which is bound to saturated or unsaturated, straight or branched $C_{1-4}$ alkylene optionally interrupted with a carbonyl group; and Z is a functional material [hereinafter referred to as the compound (2)], or a salt thereof.

The present invention further provides a metal complex compound wherein the compound (2) is chelated with at least one metal ion selected from the group consisting of ions of the elements having atomic number of 31, 39, 43, 44, 49, 57 to 71, 76, 77, 79, 81 and 83.

Further, the present invention also provides a drug for MRI diagnosis, X-ray diagnosis, radiodiagnosis or radiotherapeutics comprising at least one metal complex compound of the present invention.

DETAILED DESCRIPTION OF THE INVENTION

In the compound (1), $R_1$ and $R_2$ are the same or different and are independently a hydrogen atom or a $C_{1-4}$ alkyl group. Preferably, $R_1$ is methyl group and $R_2$ is a hydrogen atom, or both $R_1$ and $R_2$ are methyl groups.

The integer of n represents the length of the methylene chain between the two nitrogen atoms shown and may be any number in so far as usefulness of a functional material to be coupled and the resulting metal complex compound is not substantially influenced. However, in order to maintain water-solubility, n should be not so large. In particular, n is 1 to 6 and, preferably 1 to 3.

The terminal reactive group of A is a group which can readily react with a reactive group in a functional material such as thiol group, amino group, carboxyl group, carbonyl group, hydroxyl group, aldehyde group, an aromatic group (e.g., phenyl, etc.) or a heterocyclic group (e.g., imidazolyl group, etc.). Examples of the terminal reactive group of A include a hydrogen atom (i.e., the terminal group of the reactive side chain is an amino group); a halogen atom such as chlorine atom, bromine atom or iodine atom; a group selected from thiol group, hydrazine group ($-NHNH_2$) and its derivative [e.g., $-NH(CH_3)NH_2$, $-NHCONHNH_2$, $-NHCSNHNH_2$, etc.]; -NCO; $-COR_3$ [$R_3$ is azide ($N_3$) group, $C_{1-4}$ alkoxy (e.g., methoxy, etc.), imidyloxy group (e.g., succinimidyloxy group, etc.) or imidazoyloxy group]; -COX" (X" is a halogen atom such as chlorine atom or bromine atom); N-hydroxysuccinimide ester and the like.

The reactive functional group represented by A has $C_{1-4}$ alkylene group optionally interrupted with a carbonyl group between the terminal reactive group and the nitrogen group to which the group A is attached (e.g., $-COCH_2-$, $-COCH_2CH_2CH_2-$, $-CH_2COCH_2CH_2-$, $-CH_2CH_2CH_2-$, $-CH_2CH_2-$, etc.).

The alkylene group may be saturated or unsaturated and straight or branched and the alkylene chain may be substituted or interrupted by the carbonyl group at any position of the alkylene chain. However, as described above, the alkylene chain should not contain any straight or branched aryl group therein. Thereby, when the resulting metal complex compound of the present invention is decomposed and metabolized in a living body, the metal complex can be rapidly excreted in a urine. Examples of A include $-COCH_2Cl$, $-COCH_2CH_2CH_2Cl$, $-CH_2COCH_2CH_2Cl$, $-CH_2CH_2CH_2Cl$, $-CH_2CH_2COCl$ and $-CH_2CH_2SH$. Preferably, A is a hydrogen atom or a haloacetyl group such as chloroacetyl or the like.

Examples of the salt of the compound (1) include neutral salts having inorganic or organic physiologically acceptable cations as counter ions of the carboxylic acids. Examples of the counter ion include inorganic cations such as sodium, potassium, lithium and the like, and organic cations such as those derived from megulmine, dimegulmine, ethanolamine, diethanolamine, morpholine, lysine, arginine, ornithine and the like.

The groups $R_1$, $R_2$, n and A' of the compound (2) are the same as those described with respect to the compound (1) and examples the salts thereof include the same salts as those described with respect to the compound (1).

Z is a functional material. The functional material to be used in the present invention is not specifically limited and, according to a particular purpose in medical imaging diagnosis and treatment, the desired functional material can be selected for synthesis of the desired compound (2) and metal complex compound. As described above, the functional material is a biological material or a material which can interact with a biological system. Examples of the preferred functional material include low molecular weight materials such as amino acids, peptides, sugars, fatty acids, steroids, porphyrins, organ-specific functional groups and the like, and high molecular weight materials such as proteins, antibodies, antibody fragments, enzymes, polysaccharides and the like.

Thus, the reactive terminal A' is coupled to the functional material Z by means of a covalent bond.

The complex compound (2) and its metal complex of the present invention can contain at least one molecular of the compound (1) coupled with one molecular of any functional material, and its metal complex.

The metal ion which forms the complex with the compound (2) of the present invention is selected from the group consisting of ions of the elements having atomic number of 31, 39, 43, 44, 49, 57-71, 76, 77, 79, 81 and 83 according to a particular imaging diagnosis or radiotherapeutics. Normally, a trivalent (+3) metal ion is preferred.

When using the metal complex compound of the present invention is used in MRI diagnosis, the metal ion should be paramagnetic and it is selected from the group consisting of ions of lanthanide elements having atomic number of 50-70. Preferably, the metal ion is Gd, Dy or Ho ion.

In the case of using it in X-ray diagnosis, the metal ion is selected from the group consisting of ions of lanthanide elements having atomic number of 50-70 and ions of the elements having atomic number of 76 and 83. Preferably, the metal ion is Bi or Os ion.

Further, in the case of using it in radodiagnosis, the metal ion should be radioactive and, suitably, it is ions of In, Ga, Tc, Y, Ge or Sm. In the case of using it in radiotherapeutics, the radioactive metal ion of Y, Sm or Bi is selected.

For the production of the 1-substituted-1, 4, 7, 10-tetraazacyclododecane-4, 7, 10-triacetic acid type bifunctional complexing agent of the present invention represented by the formula (1), it is desirable to obtain as an intermediate compound a compound of the formula:

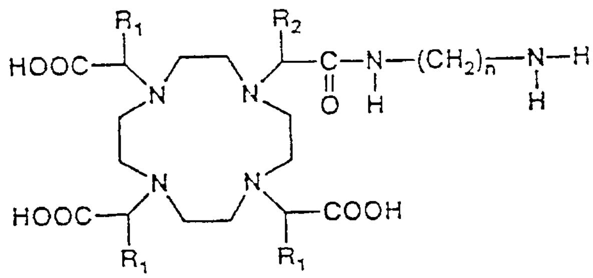

(3)

wherein n, $R_1$ and $R_2$ are as defined above, which is also useful as the bifunctional complexing agent and corresponds to the compound (1) wherein A is a hydrogen atom [hereinafter sometimes referred to as the compound (3)]. By using the compound (3) as an intermediate, various bifunctional complexing agents having various reactive groups which can be applicable to various coupling reactions with functional materials.

A preferred process for the production of the compound (3) is generally illustrated as follows:

Namely, the reactive functional side chain moiety of the compound (3) is reacted with a known compound, 1, 4, 7, 10-tetraazacyclododecane (hereinafter abbreviated as DOT) of the formula:

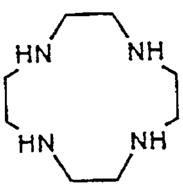

(4)

to obtain the desired 1-substituted-DOT. Then, carboxylic acid moieties are introduced to the remaining three nitrogen atoms to obtain the desired intermediate compound (3) which is also the bifunctional complexing agent.

Although a commercially available DOT is generally in the form of a salt, it is desirable to subject it to desalting treatment. Normally, the desalting treatment is carried out by using an ion exchange resin.

The reactive functional side chain moiety of the compound (3) can be produced, for example, by reacting a reactive carboxylic acid derivative of the formula:

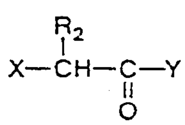

(5)

wherein $R_2$ are as defined above; X is a halogen atom; and Y is a halogen atom, a hydroxyl group or a $C_{1-4}$ alkoxy group [hereinafter referred to as the compound (5)], with a diamine compound of the formula:

$H_2N\text{-}(CH_2)_n\text{-}NH_2$ (6)

wherein n is as defined above [hereinafter referred to as the compound (6)], in the presence of a base such as triethylamine or the like. This reaction can be carried out by a per se known process. For example, the reaction of the compound (5) wherein Y is a halogen atom or an alkoxy group with the compound (6) can be carried out according to a process disclosed in the literature [when Y of the compound (5) is a halogen

atom, Fourie, P.J. et al., Eur. J. Nucl. Med., 4, 445 (1979); when Y of the compound (5) is an alkoxy group, Washburn, L.C., Nucl. Med. Biol., 18, 313 (1991)]. In addition, one of the amino groups of the diamine compound (6) may be protected with a known amino protecting group. The protecting group is finally deprotected, for example, with an acid catalyst to obtain the desired compound (3).

The introduction of the reactive functional side chain moiety thus obtained to the nitrogen atom of the DOT ring can be carried out according to a known technique.

The introduction of the carboxylic acid moiety to the remaining three nitrogen atoms in the DOT ring can be carried out by alkylation with a halocarboxylic acid derivative of the formula:

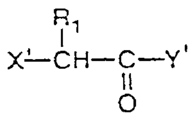

(7)

wherein $R_1$ as defined above; X' is a halogen atom; and Y' is hydroxyl group or a $C_{1-4}$ alkoxy group [hereinafter referred to as the compound (7)]. When the compound (7) wherein Y' is hydroxyl group is used, preferably, the alkylation is carried out in water at pH about 9-10. The pH can be adjusted and maintained with an inorganic base such as an alkali metal hydroxide or the like and the reaction system is warmed at about 50-80 °C. When the compound (7) wherein Y' is a $C_{1-4}$ alkoxy group is used, preferably, the alkylation is carried out in an organic solvent such as acetonitrile, tetrahydrofuran, dimethylformamide or the like at a high temperature, for example, at reflux temperature (EP-A 0 353 450). However, in this case, saponification is required.

The introduction of the carboxylic acid moieties to the nitrogen atoms of the DOT ring may be carried out prior to the introduction of the reactive functional side chain moiety. In this case, the nitrogen atom to which the reactive functional side chain is introduced is suitably protected, for example, with benzyl group or the like (JP-A 63-41468).

For example, the compound (1) wherein $R_1$ is $CH_3$, $R_2$ is H, n is 2 and A is H can be produced as follows:

Firstly, one amino group of ethylenediamine is protected with t-butoxy carbonate according to a per se known technique and then reacted with chloroacetyl chloride in ether with ice-cooling to obtain (1-butoxycarbonyl)-aminoethylcarbamoylmethyl chloride. Then, (1-butoxycarbonyl)-aminoethylcarbamoylmethyl chloride is reacted with DOT which has been subjected to desalting treatment in acetonitrile under reflux to introduce the reactive functional side chain moiety. Finally, the resultant is reacted with ethyl 2-buromopropionate in acetonitrile in the presence of potassium carbonate to obtain the corresponding triester compound and the triester compound is subjected to deprotection of the protecting group, t-butoxycarbonyl and hydrolysis of the ester groups with an acid solvent to obtain the desired bifunctional complexing agent of the compound (1).

Likewise, when the compound (1) wherein $R_1$ is a hydrogen atom or an alkyl group other than methyl is required, the desired compound can be obtained by using monobromoacetic acid, 2-bromobutanoic acid, 2-bromovaleric acid or an ester derivative thereof instead of ethyl 2-bromopropionate (in the case of the ester derivative, saponification is required). When the compound (1) wherein $R_2$ is a $C_{1-4}$ alkyl group is required, the desired compound can be obtained by using 2-chloropropionyl chloride, 2-bromobutylyl bromide, ethyl 2-bromovalerate, bromohexanoyl bromide or the like instead of chloroacetyl chloride.

The compound (1) wherein A is a hydrogen atom itself is useful as a bifunctional complexing agent. However, as described above, it can be used as an intermediate for producing the other bifunctional complexing agents of the present invention having various reactive terminal groups. For example, the terminal amino group of the reactive functional side chain moiety of the compound (1) wherein A is a hydrogen atom can be reacted with chloroacetyl chloride in anhydrous dimethylformamide at room temperature to obtain the compound (1) wherein $R_1$ is $CH_3$, $R_2$ is H, n is 2 and A is $COCH_2Cl$. According to the same manner, the desired compounds (1) having various terminal reactive groups in the side chain moieties A such as a halogen atom, thiol group, hydrazine group and a derivative thereof, -NCO, $-COOR_3$ - (wherein $R_3$ is a $C_{1-4}$ alkyl group), aldehyde group, -COX (wherein X is a halogen atom such as chlorine, bromine, etc.) and N-hydroxysuccinimide ester group can be obtained.

Coupling of the bifunctional complexing agent, the compound (1), with the desired functional material can be carried out by reaction of the terminal reactive group in the side chain moiety of the compound (1) with a nucleophilic group of the functional material such as thiol group, amino group, carboxyl group, carbonyl group, hydroxyl group, aldehyde group, an aromatic group (e.g., phenyl, etc.), a heterocyclic group

(e.g., imidazolyl group, etc.) or the like. For example, the reaction of the compound (1) with an antibody can be carried out in an aqueous solvent such as a phosphate buffer solution or the like under cooling to gentle warming (e.g., at room temperature) with stirring for several minutes to several hours (e.g., for 30-60 minutes). If necessary, prior to the coupling reaction, the functional material can be subjected to a suitable treatment in order to form a suitable reactive group for the reaction with the compound (1). For example, in the case of a sugar, it can be firstly activated with cyanogen bromide and then reacted with the compound (1) having an amino group as the reactive group to obtain the desired complex compound (2).

The salt of the compound (1) or (2) can be prepared according to a per se known technique, for example, a reaction with a suitable base or acid in a suitable aqueous solvent.

A metal corresponding to the desired metal ion is not specifically limited and can be suitably selected by a person skilled in the art. Such a metal may be the metal itself or an inorganic compound thereof (e.g., a chloride, an oxide, etc.).

Complexing can be carried out in a weak acidic to weak basic aqueous solution at, preferably, pH about 4-9. The reaction temperature is suitably selected from the temperature range of 0 to 100 °C. The amount of the metal ion to be used is from a trace amount to equimolar amount of the bifunctional complexing agent or more. The complexing can be carried out prior to or after the coupling of the compound (1). This order can be suitably selected so that denaturation of the functional material to be coupled is avoided.

The physiologically acceptable metal complex compound of the present invention thus obtained can be mixed with any pharmaceutically acceptable additives according to a conventional manner to prepare an imaging agent for diagnosis in any suitable form. Preferably it can be formulated in the form of a solution drug for diagnosis or treatment by dissolving it in a physiologically acceptable aqueous solvent.

For using the metal complex compound of the present invention as a drug for MRI diagnosis, in general, the metal complex compound can be administered in an amount of 0.0001-10 mmol/kg, preferably 0.005-0.5 mmol/kg in terms of the amount of metal ion. For using the metal complex compound of the present invention as a drug for X-ray diagnosis, the metal complex compound can be administered in an amount of 0.01-20 mmol/kg, preferably 0.1-10 mmol/kg in terms of the amount of metal ion. Further, for using the metal complex compound of the present invention as a drug for radiodiagnosis or radiotherapeutics, it can be administered in an amount of 370-18500 MBq in terms of radioactivity. Normally, the metal complex compound is administered intravenously. However, depending upon a particular situation, it can be administered orally or intraarterially.

The following Examples and Test further illustrate the present invention in detail but are not to be construed to limit the scope thereof. The abbreviations used hereinafter have the following meaning:

DO3MA: 1, 4, 7, 10-tetraazacyclododecane-1-aminoethylcarbamoylmethyl-4,7, 10-tris[(R,S)-methylacetic acid]

ClDO3MA: 1, 4, 7, 10-tetraazacyclododecane-[(N-chloroacetyl)aminoethylcarbamoylmethyl]-4, 7, 10-tris-[(R,S)-methylacetic acid]

DNS-DO3MA: 1, 4, 7, 10-tetraazacyclododecane-[(N-dansyl)aminoethylcarbamoylmethyl]-4, 7, 10-tris[-(R,S)-methylacetic acid]

DOT: 1, 4, 7, 10-tetraazacyclododecane

Example 1

Synthesis of DO3MA

a) Synthesis of N-(t-botoxycarbonyl)ethylenediamine

Ethylenediamine (270 ml, 4.04 mol) was slowly added dropwise to methanol (500 ml) with cooling in ice-water. To the mixture was added a solution of di-t-butyl-dicarbonate (100 g, 0.46 mol) in methanol (140 ml) dropwise by portions over 2 hours and the mixture was stirred at room temperature overnight. Insoluble materials were filtered off and the filtrate was concentrated. To the residue was added water and the mixture was stirred at room temperature for 30 minutes. Insoluble materials were filtered off and the filtrate was concentrated and extracted twice with acetic acid. The organic phases were combined, dried over anhydrous sodium sulfate, concentrated and distilled off under reduced pressure to obtain oil of the desired product (56.5 g, yield: 77%).

b) Synthesis of (t-butoxycarbonyl)aminoethylcarbamoylmethyl chloride

N-(t-Butoxycarbonyl)ethylenediamine ( 56.5 g, 0.35 mol) was dissolved in ether (300 ml). Under cooling with ice-water, triethylamine (70.8 g, 0.70 mol) was added and then to the mixture was added chloroacetyl chloride (39.6 g, 0.35 mol) dissolved in ether (300 ml) dropwise by portions over 3.5 hours. The mixture was stirred at room temperature for 2 hours. The precipitated crystals were filtered off. Water (300 ml) was added and the mixture was stirred at room temperature. Again, the crystals were filtered off, dissolved in methylene chloride (600 ml) and washed twice with water. The organic phases were combined, dried over anhydrous sodium sulfate, concentrated under reduced pressure to obtain crystals of the desired product (50.2 g, yield: 61%).

c) Synthesis of 1-[(t-butoxycarbonyl)aminoethylcarbamoylmethyl]-1, 4, 7, 10-tetraazacyclododecane

DOT tetrahydrochloride (23 g) was subjected to desalting by passing it through an ion exchange column (strong basic resin: DIAION SA20A) to obtain DOT (13 g, 75.5 mmol). To DOT was added acetonitrile (720 ml) and the mixture was dissolved under reflux. To the solution was added (t-butoxycarbonyl)aminoethylcarbamoylymethyl chloride (8.6 g, 36.3 mmol) dissolved in acetonitrile (180 ml) dropwise by portions over 3 hours and the mixture was stirred under reflux for 3 hours and further stirred at room temperature over night. The precipitated materials were filtered off and the filtrate was concentrated and dried under reduced pressure to obtain oil (20.6 g). The resultant was subjected to desalting treatment by passing it through an ion exchange column (strong basic resin: DIAION SA20A) and the eluate was concentrated. The residue was purified by silica gel column chromatography (eluent: chloroform:methanol: aqueous ammonia-8:4:1) to obtain crystals of the desired product (10.0 g, yield: 74%).

d) Synthesis of 1, 4, 7, 10-tetraazacyclododecane-1-aminoethylcarbamoylmethyl-4, 7, 10-tris[(R,S)-methylacetic acid] triethyl ester

1-[(t-Butoxycarbonyl)aminoethylcarbamoylmethyl]-1, 4, 7, 10-tetraazacyclododecane (8.2 g, 22.0 mmol) was dissolved in acetonitrile (800 ml) and potassium carbonate 32.8 g, 0.237 mol) was suspended in the solution. Then, ethyl 2- bromopropionate (47.6 g, 0.263 mol) was added and the mixture was stirred under reflux for 7 hours. Insoluble materials were filtered off and the filtrate was concentrated. The residue was purified by silica gel column chromatography (eluent: methylene chloride:methanol = 9:1) to obtain the desired product (12.0 g, yield: 81%).

e) Synthesis of DO3MA hydrochloride

To 1, 4, 7, 10-tetraazacyclododecane-1-aminoethylcarbamoylmethyl-4, 7, 10-tris[(R,S)-methylacetice acid] triethylester (12.0 g, 17.8 mmol) was added acetic acid (100 ml) and the mixture was stirred at room temperature. Conc. hydrochloric acid (50 ml) was added by portions thereto and the mixture was stirred at room temperature for 1 hour. Then, water (100 ml) was added and the mixture was refluxed for 4 hours and then concentrated. The residue was dissolved in water (100 ml) and the solution was treated with active charcoal and filtered off. The filtrate was concentrated and dried under reduced pressure to obtain pale yellow crystals of the desired product (9.4 g, yield: 100%).

$^1$H-NMR spectrum ($D_2O$, 270 MHz, $\delta$ ppm): 1.2-1.7 (9H, m), 2.8-3.7 (22H, m), 4.2-4.6 (3H, m).

FAB-mass spectrum ($^+$, m/z): 499.5[$(M+H)^+$], 511.5 [$(M+Na)^+$], 533.5 [$(M+2Na-H)^+$].

IR spectrum (KBr): 1400 ($CH_2$), 1480(CONH), 1620 ($NH_2$), 1680 (COOH) $cm^{-1}$.

Example 2

Synthesis of ClDO3MA

DO3MA (2.0 g, 4 mmol) was dissolved in anhydrous dimethylformamide (20 ml) and triethylamine (1.67 ml, 12 mmol) was added thereto. To the mixture was added chloroacetyl chloride (0.64 ml, 8 mmol) and the reaction was carried out at room temperature for 24 hours. The precipitate was filtered off and the filtrate was distilled off. The resulting oil was dissolved in water and the solution was stirred at room temperature for 1 hour. This solution was concentrated and passed through an ion exchange column (strong basic resin: AG1-X4, eluent: water). The eluate was concentrated and, again, passed through an ion exchange column (strong acidic resin: AG50W-X8, eluent: water). The eluate was evaporated to dryness to obtain pale yellow

semi-crystals of the desired product (1.24 g, yield: 505).

$^1H$-NMR spectrum ($D_2O$, 270 MHz, δ ppm): 1.2-1.7 (9H, m), 2.8 (2H, s), 3.0 (2H, s), 3.1-3.7 (18H, m), 4.2 (2H, s), 4.3 (3H, s).

FAB-mass spectrum ($^+$, m/z): 565 [((M + H)$^+$].

IR spectrum (KBr): 1240 ($CH_2Cl$), 1400 ($CH_2$), 1460 and 1550 (CONH), 1660 (COOH) $cm^{-1}$.

Example 3

Synthesis of DO3MA-Gd

DO3MA (1.49 g, 2.8 mmol) was dissolved in water (15 ml) and gadolinium oxide (506.5 mg, 1.4 mmol) was added thereto. The mixture was adjusted to pH 8.0-9.0 by addition of a suitable amount of an aqueous sodium oxide solution and the solution was reacted at 60 °C for 24 hours. The precipitate formed was filtered off and the filtrate was concentrated. The residue was dissolved in water and passed through an ion exchange column (strong acidic resin: AG50W-X8, eluent: 1M aqueous ammonia). The eluate was concentrated to obtain pale yellow crystals of the desired product (1.04 g, yield: 57%).

FAB-mass spectrum ($^+$, m/z): 644 [(M + H)$^+$].

IR spectrum (KBr): 1390 ($CH_2$), 1452 (CONH), 1601 ($NH_2$, $COO^-$) $cm^{-1}$.

Example 4

Synthesis of Cl-DO3MA-Gd

DO3MA-Gd (642.9 mg, 1 mmol) was suspended in anhydrous dimethylformamide (20 ml) and triethylamine (0.14 ml, 1 mmol) was added thereto. To the mixture was added dropwise chloroacetyl chloride (0.4 ml, 5 mmol) and the mixture was reacted with stirring at room temperature for 24 hours. The reaction mixture was concentrated and the residue was dissolved in water. Active charcoal was added to the solution and the mixture was stirred overnight and then filtered off. The filtrate was concentrated and passed through an ion exchange column (strong basic resin: AG1-X4, eluent: water). The eluate was concentrated to obtain white crystals of the desired product (767 mg, yield: 100%).

FAB-mass spectrum ($^+$, m/z): 720 [(M + H)$^+$].

IR spectrum (KBr): 1300 ($CH_2Cl$), 1360 ($CH_2$), 1450 and 1490 (CONH), 1600 ($COO^-$) $cm^{-1}$.

Example 5

Synthesis of DO3MA-In-111

DO3MA (20 mg, 0.038 mmol) was dissolved in water and pH was adjusted to 8.5 by addition of 0.5N sodium hydroxide solution. One ml portion of this solution was taken and to this were added indium chloride solution (In-111, 1280 MBq, 0.1 ml) and then 1N sodium hydroxide solution (0.1 ml) and the mixture was reacted under pressurized steam (1 kg/$cm^2$) for 10 minutes. The resulting reaction mixture was passed through a cation exchange cartridge (Seppack, eluent: water) to obtain DO3MA-In-111.

Radiochemical purity (TLC): 96.2% (Rf: 0.40).

Example 6

Synthesis of DO3MA-Bi

DO3MA (105 mg, 0.2 mmol) was dissolved in water and to the solution was added bismuth chloride ( 69 mg, 0.22 mmol). The mixture was adjusted to pH 8.0 by addition of a suitable amount of sodium hydroxide solution. The resulting solution was reacted with stirring at 60 °C for 24 hours. Insoluble materials were filtered off and the filtrate was concentrated to obtain white powder of the desired product (156 mg, yield 100%).

$^1H$-NMR spectrum ($D_2O$, 270 MHz, δ ppm): 1.4 (6H, m), 1.7 (3H, m), 2.9 (2H, m), 3.2 (6H, m), 3.5 (8H, m), 3.8 (4H, m), 4.1 (5H, m).

FAB-mass spectrum ($^+$, m/z): 695 [(M + H)$^+$], 717[(M + Na-H)$^+$].

IR spectrum (KBr): 1387 ($CH_2$), 1450 (CONH), 1593 ($NH_2$, $COO^-$) $cm^{-1}$.

Example 7

Synthesis of DNS-DO3MA-Gd

DO3MA-Gd (1.323 g, 2.1 mmol) was suspended in anhydrous dimethylformamide (20 ml) and to the suspension was added dropwise dansyl chloride (2.832 g, 10.5 mmol) dissolved in anhydrous dimethylformamide (5 ml). Further, triethylamine (0.32 ml, 2.3 mmol) was added and the mixture was reacted at room temperature for 24 hours. The reaction mixture was filtered and the filtrate was concentrated. Water was added and the mixture was washed with ethyl acetate. Aqueous phases were combined, adjusted to pH 8, treated with active charcoal and concentrated. The residue was purified by subjecting to silica gel column chromatography (silica gel 60, eluent: chloroform:methanol:aqueous ammonia = 2:2:1) to obtain fluorescent orange powdery crystals of the desired product (72.5 mg, yield: 4%).

IR spectrum (KBr); 795 (Ar-H), 1144 and 1389 ($SO_2NH$), 1456 ($CH_2$), 1620 ($COO^-$) $cm^{-1}$.

FAB-mass spectrum ($^+$, m/z): 877 [$(M+H)^+$].

Example 8

Hepatic MRI by using DNS-DO3MA-Gd

DNS-DO3MA-Gd solution (Gd concentration: 10 mmol) was administered to ICR female mouse (30 g, 7 week old) anesthetized with thiopental through the tail vein at a dose of 50 mmol/kg. Immediately after administration, the mouse was fixed in the prone position in the magnetic field of a MRI apparatus and imaging of a traverse view of the abdominal region including the liver was carried out. As a control, before administration of the compound of the present invention, the same animal was subjected to the MRI of the same region to obtain a control image.

The apparatus used was CSI Omega (General Electric) and imaging was carried out under the following conditions: magnetic field intensity: 2T; imaging coil: 4.5 cm$\phi$ bird-cage type coil; spin echo technique with a slice thickness of 4 mm, integrating 4 times and a resolving power of 256 x 256; and T1 weighted (TR/TE: 500/100 msec).

Fig. 1 is a photograph showing the traversal view of the control, i.e., before administration of the compound of the present invention. Fig. 2 is a photograph showing the same traversal view at 55 minutes after administration of the DNS-DO3MA-Gd solution. Dansyl group is a functional group having directional characteristics toward the liver (Japanese Patent Appln. No. 3-291260) and is excreted from blood circulatory system through the liver to the intestines. Signal intensity of the mouse liver is enhanced by the compound of the present invention and it has been found that the behavior of the bifunctional complexing agent of the present invention in a living body is clearly controlled by the functional material.

Test 1

Stability of DO3Ma-Gd in serum

DO3MA-Gd (24.0 mg, 37 mmol) was made up to 10 ml with $H_2O$, accurately to obtain 3.7 mM solution. Serum (0.18 ml) obtained from Sprague-Dawley female rat (14 week old) was added thereto to obtain a sample solution. As a control, the rat serum (0.18 ml) was added to $H_2O$ (0.02 ml) to obtain a control solution.

Immediately after preparation, the above sample and control solutions were incubated in a water bath at 37 °C. T1 relaxation time of each solution was determined at 37 °C using NMR (0.5T, FSE-60 pulse NMR apparatus, manufactured by Nihondenshi K.K., Japan) at 5 minutes and 1, 3, 6 and 24 hours after preparation of the solutions.

As a result, in the case of the sample solution, T1 relaxation times were 363 msec. and 343 msec. at 5 minutes and 24 hours after the preparation, respectively and the relaxation time was somewhat shortened. On the other hand, in the case of the control solutio, T1 relaxation times were 1970 msec. and 1870 msec. at 5 minutes and 24 hours after the preparation, respectively and the relaxation time was shortened by 100 msec. The results are shown in Table 1.

Table 1

| Relaxation time of DO3MA-Gd (T1, msec.) | | |
|---|---|---|
| Time (hrs.) | Sample sol. | Control sol. |
| 0.083 | 363.1 | 1970 |
| 1 | 363.2 | 1930 |
| 3 | 356.4 | 1920 |
| 6 | 343.2 | 1860 |
| 24 | 342.6 | 1870 |

Then, the ratio of the sample and the control (S/C, %) at each determination time was calculated. As a result, S/C at 5 minutes after preparation was 18.4% and S/C at 24 hours after preparation was 18.3% and no substantial difference was observed. The results of this calculation are shown in Table 2.

Table 2

| Ratio of relaxation times of DO3MA-Gd and control (S/C) | |
|---|---|
| Time (hrs.) | S/C(%) |
| 0.083 | 18.4 |
| 1 | 18.8 |
| 3 | 18.5 |
| 6 | 18.4 |
| 24 | 18.3 |

If DO3MA-Gd were instable in serum and Gd were readily released, great shortening of the relaxation time due to free Gd should be caused. However, even if 24 hours had been lapsed, no substantial shortening of the relaxation time of DO3MA-Gd was observed. Thus, it is clear that complex stability of the compound of the present invention in serum is very high.

As described hereinabove, the present invention provides the novel DO3A type cyclic bifunctional complexing agent which can form the metal complex having high stability, good solubility due to no charge, great physiological acceptability and the reactive functional terminal group which can readily be coupled with the functional material. Further, the present invention provides the complex compound wherein the novel bifunctional complexing agent is coupled to the functional material as well as the metal complex wherein the complex compound is coupled to the metal ion. The metal complex of the present invention can be used as a drug for MRI diagnosis, X-ray diagnosis, radiodiagnosis and radiotherapeutics.

## Claims

**1.** A physiologically acceptable compound of the formula:

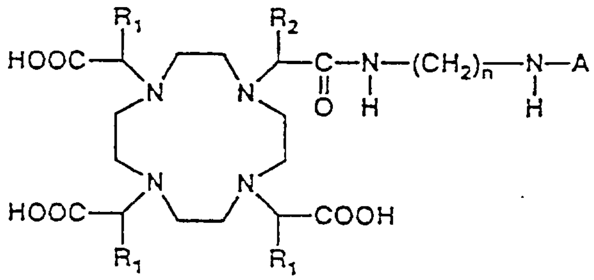

(1)

wherein n is an integer of 1 to 6; $R_1$ and $R_2$ are the same or different and are independently an hydrogen atom or a $C_{1-4}$ alkyl group; and A is an hydrogen atom or a reactive functional group having a terminal reactive group which is bound to a saturated or unsaturated, straight or branched $C_{1-4}$

alkylene group optionally interrupted with a carbonyl group, or a salt thereof.

**2.** A compound according to claim 1, wherein the terminal reactive group is a halogen atom, thiol group, hydrazine group or a derivative thereof, -NCO, -COOR$_3$ (wherein R$_3$ is C$_{1-4}$ alkyl), aldehyde group, -COX" (wherein X" is a halogen atom) or N-hydroxysuccinimide ester group.

**3.** A compound according to claim 1, wherein R$_1$ is methyl, R$_2$ is hydrogen and n is an integer of 1-3.

**4.** A compound according to claim 1, wherein R$_1$ and R$_2$ are methyl and n is 1-3.

**5.** A compound according to claim 1, wherein A is hydrogen.

**6.** A compound according to claim 1, wherein A is haloacetyl.

**7.** A physiologically acceptable complex compound of the formula:

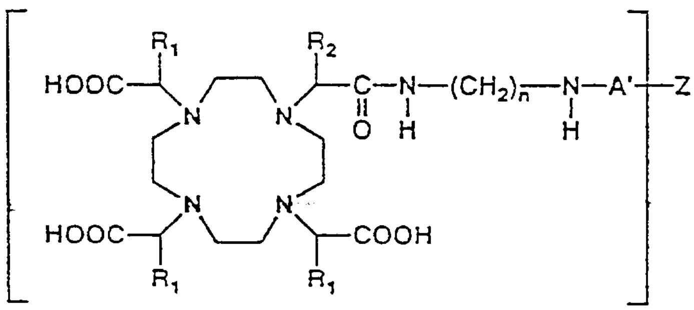

(2)

wherein n is an integer of 1 to 6; R$_1$ and R$_2$ are the same or different and are independently a hydrogen atom or a C$_{1-4}$ alkyl group; A' is a reactive functional residue having a terminal reactive group which is bound to saturated or unsaturated, straight or branched C$_{1-4}$ alkylene optionally interrupted with a carbonyl group; and Z is a functional material, or a salt thereof.

**8.** A metal complex compound chelated with at least one metal ion selected from the group consisting of ions of the elements having atomic number of 31, 39, 43, 44, 49, 57 to 71, 76, 77, 79, 81 and 83.

**9.** A metal complex compound according to claim 8, wherein the metal ion is Gd, Dy, Ho, Bi, Os, In, Ga, Tc, Y or Sm.

**10.** A drug for nuclear magnetic resonance imaging diagnosis, X-raydiagnosis, radiodiagnosis or radiotherapeutics comprising at least one metal complex compound according to claim 8.

**11.** A drug according to claim 10, wherein the metal complex compound is that according to claim 9.

Fig. 1

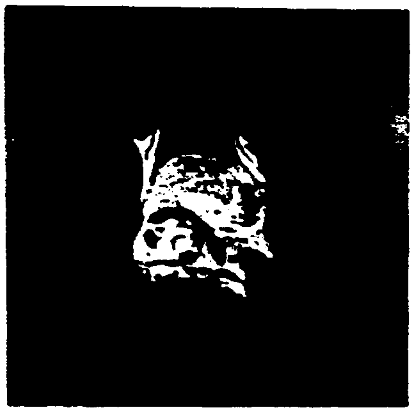

Fig. 2

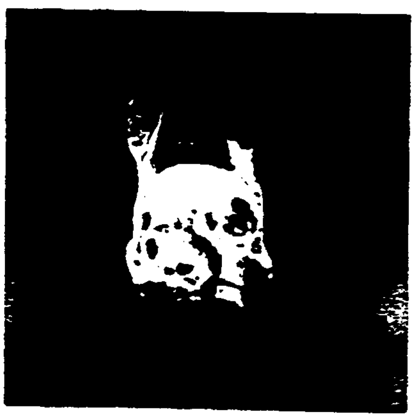

European Patent Office

# PARTIAL EUROPEAN SEARCH REPORT

which under Rule 45 of the European Patent Convention shall be considered, for the purposes of subsequent proceedings, as the European search report

Application Number: EP 93 10 5021

Page 1

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 5) |
|---|---|---|---|
| X | WO-A-9 105 762 (SALUTAR INC.) 2 May 1991 * example 34; Claim 28 (q), (s), (t) * | 1,2 | C07D257/02 A61K49/00 |
| Y | * entire document * --- | 1-6 | |
| X | EP-A-0 382 583 (CELLTECH LTD.) 16 August 1990 * claims 1-11,13 * --- | 1-6 | |
| X | EP-A-0 326 226 (NYCOMED AS) 2 August 1989 * claims 1-3 * | 1,5 | |
| Y | * entire document * --- | 1-6 | |
| Y | WO-A-9 012 050 (SALUTAR INC.) 18 October 1990 * entire document; esp. example 2, 4 , 5 * --- | 1-6 | |
| X | US-A-5 053 503 (CENTOCOR) 1 October 1991 * column 3, line 7 - column 4, line 3; claims 1-5 * --- | 1-6 | |
| | -/-- | | |

TECHNICAL FIELDS SEARCHED (Int. Cl. 5)

C07D
A61K

## INCOMPLETE SEARCH

The Search Division considers that the present European patent application does not comply with the provisions of the European Patent Convention to such an extent that it is not possible to carry out a meaningful search into the state of the art on the basis of some of the claims

Claims searched completely :

Claims searched incompletely :

Claims not searched :

Reason for the limitation of the search:

see sheet C

| Place of search | Date of completion of the search | Examiner |
|---|---|---|
| MUNICH | 29 JULY 1993 | HERZ C.P. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent Office

# PARTIAL EUROPEAN SEARCH REPORT

Application Number

EP 93 10 5021



## DOCUMENTS CONSIDERED TO BE RELEVANT

| Category | Citation of document with indication, where appropriate, of relevant passages | Relevant to claim | CLASSIFICATION OF THE APPLICATION (Int. Cl. 5) |
| --- | --- | --- | --- |
| Y | WO-A-8 912 631 (THE DOW CHEMICAL CO.) 28 December 1989 * entire document * --- | 1-6 | |
| A | EP-A-0 255 471 (SCHERING AG) 28 July 1986 * entire document * --- | 1-6 | |
| A | WO-A-8 905 802 (BRACCO INDUSTRIA CHIMICA) 29 June 1989 * entire document * --- | 1-6 | |
| A | EP-A-0 184 899 (NYEGAARD & CO.) 18 June 1986 * entire document * --- | 1-6 | TECHNICAL FIELDS SEARCHED (Int. Cl. 5) |
| A | EP-A-0 292 689 (E.R. SQUIBB & SONS, INC.) 30 November 1988 * entire document * --- | 1-6 | |
| Y | INORG. CHEM. vol. 30, no. 6, 1991, pages 1265 - 1269 DISCHINO ET AL. 'Synthesis of Nonionic Gadolinium Chelates Useful as Contrast Agents for Magnetic Resonance Imaging. 1,4,7-Tris(carboxymethyl)-10-substituted-1,4,7,10-tetraazacyclododecanes and Their Corresponding Gadolinium Chelates' * examples 16,17,19 * --- | 1-6 | |
| Y | INORG. CHEM. vol. 31, no. 12, 1992, pages 2422 - 2428 AIME ET AL. 'Synthesis, Characterization, and 1/T1 NMRD Profiles of Gadolinium(III) Complexes of Monoamide Derivatives of DOTA-like Ligands. X-ray Structure of the 10-[2-[[2-Hydroxy-1-(hydroxymethyl)ethyl]amino]-1-[(phenylmethoxy)methyl]-2-oxoethyl]-1,4,7,10-tetraazacyclododecane-1,4,7-triacetic Acid-Gadolinium' * compounds of formula 3 to 7 * --- | 1-6 | |

-/--

European Patent Office

# PARTIAL EUROPEAN SEARCH REPORT

Application Number

EP 93 10 5021



## DOCUMENTS CONSIDERED TO BE RELEVANT

| Category | Citation of document with indication, where appropriate, of relevant passages | Relevant to claim | CLASSIFICATION OF THE APPLICATION (Int. Cl. 5) |
|---|---|---|---|
| Y | BIOCONJUGATE CHEM. vol. 1, no. 1, 1990, pages 65 - 71 SIEVING ET AL. 'Preparation and Characterization of Paramagnetic Polychelates and Their Protein Conjugates' * scheme II * | 1-6 | |
| | ----- | | |

TECHNICAL FIELDS SEARCHED (Int. Cl. 5)

EP 93 105 021.5

- C -

Lack of conciseness

The definition of the following substituent(s) is too general and/or encompasses too broad a range of totally different chemical groups, only partly supported by examples given in the descriptive part of the application:

- A "reactive functional group"; "terminal reactive group"
- A' "reactive functional residue"
- Z "functional material"

- Claims 8 to 11 "metal complex compound"

The vast number of theoretically conceivable compounds resulting from the combination of all claimed substituents of the above list precludes a comprehensive search. Guided by the inventive concept as disclosed in the descriptive part of the present application the search has been limited to the following case(s):

- Claims 1 to 6 except those definitions objected to as above
- the examples

(c.f. Articles 83, 84 EPC, Rule 45 I.R., Guidelines Exam. Part B, Chapt. III, 3.6, 3.7).